# EUROPÄISCHE PATENTSCHRIFT

(11) **EP 0 623 356 B1**
(45) Veröffentlichungstag und Bekanntmachung des Hinweises auf die Patenterteilung: **02.10.1996**
(21) Anmeldenummer: 94107954.3
(22) Anmeldetag: 19.06.1991
(51) Int. Cl.: A61M 1/00, A61M 5/158, A61B 5/14, A61M 25/00

(54) **Zweilumige Entnahmenadel für Körperflüssigkeiten**
Dual lumen needle for withdrawal of body fluids
Aiguille à double lumière pour retirer les liquides du corps

(30) Priorität: 04.07.1990 AT 1425/90
(43) Veröffentlichungstag der Anmeldung: 09.11.1994
(62) Teilanmeldung aus: 91890127.3
(73) Patentinhaber: AVL Medical Instruments AG, CH-8207 Schaffhausen (CH)
(72) Erfinder: Skrabal, Falko, Prof. Dr., A-8010 Graz (AT); Kleinhappl, Erich, Ing., A-8062 Weinitzen (AT)
(74) Vertreter: Krause, Walter, Dr. Dipl.-Ing.

(56) Entgegenhaltungen:
- EP-A- 0 000 041
- EP-A- 0 191 599

## Beschreibung

Die Erfindung betrifft eine zweilumige Entnahmenadel für Körperflüssigkeiten, beispielsweise Blut oder Gewebeflüssigkeit, mit im wesentlichen parallel geführten ersten und zweiten elastischen Kanülen, wobei die erste Kanüle eine nach dem Einstechen der Entnahmenadel entfernbare Einführnadel aufweist, deren geschliffenes, spitzes oder schneidenförmiges Ende über die erste Kanüle hinausragt; wobei ein Griffteil der Entnahmenadel neben Anschlüssen für die beiden Kanülen eine durch einen elastischen Stopfen verschlossene separate Öffnung zur Entnahme der Einführnadel aufweist und beide Kanülen aus Kunststoff ausgeführt sind.

In der Medizin gibt es eine Reihe von Anwendungsgebieten für zweilumige Nadeln, die immer dann von Vorteil sind, wenn durch ein Lumen dem Körper eine Substanz zugeführt und durch das andere Lumen diese Substanz, ein Reaktionsprodukt oder eine Körperflüssigkeit entnommen werden soll.

Beispielsweise wird bei der Gewebeperfusion dem Gewebe über ein Lumen einer zweilumigen Nadel eine Perfusionsflüssigkeit zugeführt, welche nach einer kurzen Equilibrierungsphase über das andere Lumen der Nadel wieder gewonnen wird. Die Außenwand der Nadel weist dabei Öffnungen auf, um die Kontaktfläche mit dem Gewebe zu vergrößern.

So sind beispielsweise aus der WO 88/05643 mehrere Ausführungsvarianten von zweilumigen Nadeln bekanntgeworden, welche im wesentlichen aus zwei ineinanderliegenden Stahlröhrchen bestehen. Doppellumige Stahlnadeln können mit relativ geringem Gesamtdurchmesser hergestellt werden, wodurch die Schmerzbelastung des Patienten minimiert ist und weisen zudem aufgrund des verwendeten Materials die für das Einstechen in das Gewebe nötige Festigkeit auf. Nachteilig ist lediglich die Steifigkeit der Nadel, welche den Tragekomfort bei längerer Liegezeit verringert.

Aus der WO 88/05643 ist weiters ein Katheter bzw. eine Entnahmenadel aus elastischem Material bekannt, deren inneres Lumen im Querschnitt sternförmig abstehende Septen aufweist, wobei deren kapillare Zwischenräume das vom anliegenden Gewebe begrenzte äußere Lumen der Nadel bilden. Der biegsame Katheter befindet sich beim Einstechen in das Gewebe in einer Hohlnadel, welche nach dem Einstechen wieder zurückgezogen wird, sodaß nur der elastische Katheter im Gewebe verbleibt. Nachteilig bei dieser Ausführungsform ist der durch die Hohlnadel vergrößerte Gesamtdurchmesser, welcher die Schmerzbelastung des Patienten erhöht, sowie der vergrößerte Manipulationsaufwand.

Aus der EP-A 0 191 599 ist eine zweilumige Entnahmenadel bekanntgeworden, deren erste Kanüle eine nach dem Einstechen entfernbare Einführnadel aufweist. Die Entnahmenadel eignet sich allerdings nur für Dialysezwecke, da die Öffnungen der beiden Lumen axial möglichst weit voneinander entfernt sind, um zu verhindern, daß von der Dialysevorrichtung in ein Blutgefäß eingebrachtes Blut sofort wieder vom zweiten Lumen abgesaugt wird. Die zweite, einstückig mit der ersten Kanüle ausgeführte Kanüle weist Öffnungen im distalen Endbereich auf. Weiters ist ein gewisser Manipulationsaufwand erforderlich, um nach dem Entfernen der Einführnadel aus der ersten Kanüle einen Anschluß zur Dialysevorrichtung herzustellen, ohne mit Blut in Kontakt zu kommen.

Aus der EP-A 0 000 041 ist eine zweilumige Entnahmenadel für Körperflüssigkeiten der eingangs genannten Art bekannt, mit im wesentlichen parallel geführten ersten und zweiten elastischen Kanülen, wobei die erste Kanüle eine nach dem Einstechen der Entnahmenadel entfernbare Einführnadel aufweist, deren geschliffenes Ende über die erste Kanüle hinausragt. Der Griffteil der Entnahmenadel weist neben Anschlüssen für die beiden Kanülen eine durch einen elastischen Stopfen verschlossene separate Öffnung zur Entnahme der Einführnadel auf.

Aufgabe der Erfindung ist es, eine Entnahmenadel der eingangs genannten Art vorzuschlagen, welche einen geringen Gesamtdurchmesser mit einem höheren Tragekomfort bei längerer Liegezeit vereint und auch zur Gewebeperfusion verwendet werden kann. Weiters soll der Manipulationsaufwand vermindert und das Austreten von Körperflüssigkeiten verhindert werden.

Diese Aufgabe wird erfindungsgemäß dadurch gelöst, daß die zweite Kanüle einstückig mit der ersten Kanüle ausgeführt ist, wobei die Wand der ersten Kanüle in dem von der zweiten Kanüle nicht beanspruchten Bereich Wandöffnungen aufweist. Dadurch ist eine hervorragende Eignung für Anwendungen im Gewebe gegeben. Weiters kann bereits innerhalb der Entnahmenadel für eine Strömungsumkehr, beispielsweise einer Perfusionsflüssigkeit, gesorgt werden. Da im Griffteil der Entnahmenadel neben den Anschlüssen für die beiden Kanülen eine separate, durch einen elastischen Stopfen verschlossene Öffnung zur Entnahme der Einführnadel vorgesehen ist, kann auch keine Körperflüssigkeit austreten. Die für das Einstechen der Nadel benötigte Festigkeit wird durch die Einführnadel, beispielsweise aus Stahl, realisiert, wobei der Gesamtdurchmesser durch den Wegfall einer äußeren Hohlnadel klein bleibt. Nach dem Herausziehen der Einführnadel entsteht eine biegsame, elastische, zweilumige Entnahmenadel mit hohem Tragekomfort.

Eine weitere vorteilhafte Ausführungsvariante der Erfindung sieht vor, daß sich das Lumen der zweiten Kanüle erst nach dem Entfernen der Einführnadel in das Lumen der ersten Kanüle entfaltet. So lange sich die Einführnadel in der Entnahmenadel befindet, kann das Lumen der zweiten Kanüle zu einem schmalen Schlitz verformt sein, welcher sich nach dem Entfernen der Einführnadel, bedingt durch die unterschiedlichen Druckverhältnisse in den beiden Kanülen, entsprechend entfaltet. Es sind natürlich auch doppellumige Entnahmenadeln mit im wesentlichen unverformbaren Lumen denkbar.

Zur sicheren Handhabung der zweilumigen Entnahmenadel wird erfindungsgemäß vorgeschlagen, daß beide Anschlüsse verwechslungssicher in einen Kupplungsteil mit zu- und abführenden Leitungen einführbar sind.

Die Erfindung wird im folgenden anhand von Zeichnungen näher erläutert. Es zeigen:
Fig. 1 und 2 zweilumige Entnahmenadeln nach der Erfindung,
Fig. 3a und 3b Schnitte entlang der Linie II-II in Fig. 1.

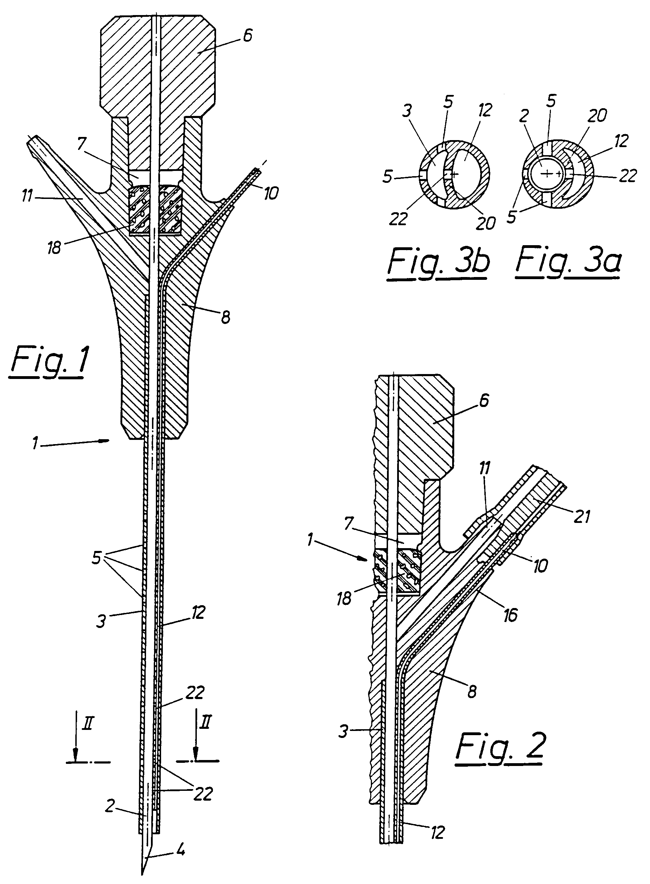

Die in Fig. 1 dargestellte Entnahmenadel 1 weist eine Einführnadel 2, z.B. eine Stahlnadel oder einen Metallstift auf, welcher von einer biegsamen ersten Kanüle 3, z.B. aus Kunststoff, eng umschlossen ist, sodaß nur das geschliffene bzw. spitz oder schneidenförmig ausgeführte Ende 4 der Einführnadel 2 frei bleibt. Die Kanüle 3 weist mehrere Wandöffnungen 5 auf, über welche die Perfusionsflüssigkeit mit dem Gewebe in Kontakt kommt. Die Einführnadel 2 ist auf der vom geschliffenen Ende 4 abgewandten Seite mit einer Halterung 6 versehen, welche einen Hohlraum 7 im Griffteil 8 der Nadel 1 verschließt. Für die Anwendung in Blutgefäßen werden Entnahmenadeln 1 verwendet, deren äußere Kanüle 3 keine Öffnungen aufweist. Im Bereich des Endes 4 der Einführnadel 2 kann die Kanüle 3 konisch an den Durchmesser der Einführnadel 2 angepaßt sein, um einen stufenlosen Übergang zu gewährleisten.

Im Hohlraum 7 des Griffteiles 8 ist ein elastischer Stopfen 18 angeordnet, welcher von der Einführnadel 2 durchsetzt wird. Nach dem Einstechen in das Gewebe wird die Einführnadel 2 an ihrer Halterung 6 herausgezogen, wonach sich die Öffnung im elastischen Stopfen 18 schließt und dieser den Hohlraum 7 automatisch abschließt. Die Entnahmenadel 1 ist dann sofort funktionsbereit.

Eine zweite Kunststoffkanüle 12 ist einstückig mit der ersten Kanüle 3 verbunden, wobei das Lumen der zweiten Kanüle in der Wand der ersten angeordnet sein kann. Dabei ist die innere Kanüle 12 deformiert und entfaltet ihr Lumen erst nach dem Herausziehen der Einführnadel 2. Die Anschlüsse 10 und 11 der Entnahmenadel 1 können direkt in den Griffteil 8 eingeformt sein und stehen mit der ersten Kanüle 3 bzw. mit der zweiten 12 in Strömungsverbindung.

Fig. 3a und 3b zeigen jeweils Schnitte im selben Bereich der Nadelspitze einmal (Fig. 3a) mit Einführnadel und einmal (Fig. 3b) nach dem Entfernen der Einführnadel, wobei sich das zuvor schlitz- oder halbmondförmige Lumen der zweiten Kanüle entfaltet.

Die Trennwand 20 zwischen erster 3 und zweiter Kanüle 12 weist in diesem Bereich Öffnungen 22 auf (siehe auch Fig. 2). Die Wandöffnungen 5 befinden sich nur in dem von der zweiten Kanüle nicht beanspruchten Bereich.

## Patentansprüche

1. Zweilumige Entnahmenadel (1) für Körperflüssigkeiten, beispielsweise Blut oder Gewebeflüssigkeit, mit im wesentlichen parallel geführten ersten und zweiten elastischen Kanülen (3, 12), wobei die erste Kanüle (3) eine nach dem Einstechen der Entnahmenadel (1) entfernbare Einführnadel (2) aufweist, deren geschllffenes, spitzes oder schneidenförmiges Ende (4) über die erste Kanüle (3) hinausragt, wobei ein Griffteil (8) der Entnahmenadel (1) neben Anschlüssen (10, 11) für die beiden Kanülen (3, 12) eine durch einen elastischen Stopfen (18) verschlossene separate Öffnung zur Entnahme der Einführnadel (2) aufweist und beide Kanülen aus Kunststoff ausgeführt sind, **dadurch gekennzeichnet**, daß die zweite Kanüle (12) einstückig mit der ersten Kanüle (3) ausgeführt ist, wobei die Wand der ersten Kanüle (3) in dem von der zweiten Kanüle (12) nicht beanspruchten Bereich Wandöffnungen (5) aufweist.

2. Entnahmenadel nach Anspruch 1, **dadurch gekennzeichnet**, daß sich das Lumen der zweiten Kanüle (12) erst nach dem Entfernen der Einführnadel (2) in das Lumen der ersten Kanüle (3) entfaltet.

3. Entnahmenadel nach Anspruch 1 oder 2, **dadurch gekennzeichnet**, daß beide Anschlüsse (10, 11) der Entnahmenadel (1) verwechslungssicher in einen Kupplungsteil (21) mit zu- und abführenden Leitungen einführbar sind.

## Claims

1. Two-channel sampling needle (1) for withdrawing body fluids, such as blood or tissue fluid, comprising two substantially parallel flexible cannulas (3, 12), the first cannula (3) being provided with an insertion needle (2) which may be removed after the sampling needle (1) has been introduced, the sharpened, pointed or knife-shaped end (4) of said insertion needle (2) projecting beyond the end of the first cannula (3), and a mounting part (8) of the sampling needle (1) being provided with fittings (10, 11) for the two cannulas (3, 12) and a separate opening for removing the insertion needle (2), which opening is plugged by an elastic stopper (18), both cannulas being made of synthetic material, **characterized in that** the second cannula (12) is configured in one piece with the first cannula (3), the wall of the first cannula (3) having perforations (5) in the area not used by the second cannula (12).

2. Sampling needle according to claim 1, **characterized in that** the lumen of the second cannula (12) extends into that of the first cannula (3) only after the insertion needle (2) has been removed.

3. Sampling needle according to claim 1 or 2, **characterized in that** both fittings (10, 11) of the sampling needle (1) can be inserted into a coupling element (21) with ingoing and outgoing lines, without any risk of confusion.

## Revendications

1. Aiguille de prélèvement (1) à deux passages pour des liquides physiologiques, par exemple du sang ou du sérum, avec une première et une seconde canule (3, 12), élastiques, essentiellement parallèles, la première canule (3) comportant une aiguille d'introduction (2) qui s'enlève après l'enfoncement de l'aiguille de prélèvement (1) dont l'extrémité (4) affûtée en forme de pointe ou d'arête coupante, dépasse de la première canule (3), et une partie formant poignée (8) de l'aiguille de prélèvement (1) présente à côté des branchements (10, 11) pour les deux canules (3, 12) une ouverture distincte fermée par un bouchon élastique (18) pour extraire l'aiguille d'introduction (2) et les deux canules sont en matière plastique, caractérisée en ce que la seconde canule (12) fait corps avec la première canule (3) et la paroi de la première canule (3) comporte des ouvertures (5) de paroi dans la plage non sollicitée par la seconde canule (12).

2. Aiguille selon la revendication 1,
caractérisée en ce que
le passage de la seconde canule (12) ne se déploie dans le passage de la première canule (3) qu'après enlèvement de l'aiguille d'introduction (2).

3. Aiguille selon la revendication 1 ou 2,
caractérisée en ce que
les deux branchements (10, 11) de l'aiguille de prélèvement (1) peuvent être introduits dans une partie d'accouplement (21) avec les conduites d'alimentation et d'évacuation d'une manière interdisant tout risque d'interchangeabilité.
